(19) Europäisches Patentamt
European Patent Office
Office européen des brevets

(11) **EP 2 295 037 A1**

(12) **EUROPEAN PATENT APPLICATION**

(43) Date of publication:
**16.03.2011 Bulletin 2011/11**

(21) Application number: **09170031.0**

(22) Date of filing: **11.09.2009**

(51) Int Cl.:
*A61K 9/16* (2006.01)     *A61K 9/20* (2006.01)
*A61K 9/28* (2006.01)     *A61K 9/48* (2006.01)
*A61K 9/26* (2006.01)     *A61K 9/32* (2006.01)
*A61K 31/7056* (2006.01)

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(71) Applicant: **Ratiopharm GmbH
89079 Ulm (DE)**

(72) Inventors:
 • **Winter, Sven
  89134, Blaustein (DE)**

 • **Scheiwe, Max-Werner
  79689, Maulburg (DE)**
 • **Genth, Maria, Dr.
  89155 Erbach (DE)**

(74) Representative: **Kalhammer, Georg et al
  Lederer & Keller
  Patentanwälte
  Prinzregentenstrasse 16
  80538 München (DE)**

(54) **Pharmaceutical formulation containing Ribavirin**

(57)    The present invention relates to a pharmaceutical dosage of ribavirin. More specifically, the present invention relates to a process and product containing a ribavirin composition and its administration.

EP 2 295 037 A1

Printed by Jouve, 75001 PARIS (FR)

**EP 2 295 037 A1**

**Description**

FIELD OF THE INVENTION

The present invention relates to a pharmaceutical dosage form of ribavirin. More specifically, the present invention relates to a process and product containing a ribavirin formulation and its administration.

BACKGROUND OF THE INVENTION

The international non-proprietary name "ribavirin" (1-β-D-Ribofuranosyl-1H-1,2,4-triazole-3-carboxamide) designates a nucleoside analogue which is approved as virusstatic for certain indications in the therapy of humans. Ribavirin is active against a number of DNA and RNA viruses. There are numbers of proposed mechanisms of action for ribavirin. These include indirect effects such as inhibition of inosine monophosphate dehydrogenase and immunomodulatory effects and direct effects such as polymerase inhibition and interference with viral RNA capping. Ribavirin has demonstrated antiviral activity *in vitro* against respiratory syncytial virus and *in vivo* in infected cotton rats when administered intraperitoneally or by aerosol.

In principle, ribavirin is active against a broad spectrum of viruses (e.g. hepatitis, influenza, measles, herpes, AIDS). Therapeutically relevant are currently only its activities against the respiratory-syncytial-virus (RSV) and the hepatitis C-virus (HCV). In Germany, ribavirin was initially approved exclusively for aerosol therapy of most severe bronchopulmonal infections caused by RSV. A ribavirin-containing dry substance for the production of solutions for inhalation is available since 1993 under the trademark "Virazole®" (ICN).

In the meantime, ribavirin was approved as "Rebetol®" (Schering-Plough/Essex Pharma) in peroral application forms for the therapy of chronic hepatitis C in combination with the cytokine interferon α-2b ("Intron A®", Schering-Plough/Essex Pharma) and interferon α-2a ("Roferon A®", Roche/Genentech) and its pegylated forms ("PegIntron®", Schering-Plough/Essex Pharma) and ("Pegasys®", Roche). The standard forms of interferon have been more and more replaced by pegylated forms because the pegylated forms provide a more constant level of interferon α in the blood. Ribavirin alone has only little effect on hepatitis C virus but in combination with interferon α the sustained reponse rate is increased by two- to three-fold. For this reason combination therapy is now successfully used in patients with HCV-infection, although the treatment duration (24 - 48-week course) and the optimal treatment dose (800 mg to 1,400 mg) appears to vary depending on the virus-genotype and the body weight of the patient.

Ribavirin compositions are also known as, for example, disclosed in U. S. Patent No. 6,337,090; Canadian Patent No. 2,135,669; and U. S. Patent Application Publication 2003/0104050 A1. As noted in U. S. Patent No. 6,337,090, there are many processing difficulties in preparing ribavirin compositions, including flowability, uniformity, etc. Ribavirin is known to have poor flow, which adversely affects its manufacture into solid dosage forms. A drug's manufacturing characteristic is important because it can affect weight variability, uniformity etc. of a dosage form of the drug, which in turns affects the drug's dissolution and bio-availability in a subject having ingested it.

EP 991 415 B1 discloses an orally administrable solid dosage form comprising a ribavirin compacted composition having a tapped density of at least 0.6 g/cm$^3$. EP 991 415 B1 further teaches that a ribavirin composition with the indicated tapped density of at least 0.6 g/cm$^3$ enables the uniform filling of 200 mg capsules. Further potential advantages of a high tapped density above 0.6 g/ml are mentioned.

EP 1 455 801 describes a process for the production of a ribavirin containing granulate, comprising granulating the ribavirin powder with a granulation solution comprising 65 % to 85 % by weight of ethanol or isopropanol. This high content of alcohol has the disadvantages that it is more laborious to recover the solvent and that the risk of residual alcohol being present in the tablet is increased.

US 7,538,094 B2 and WO 2004/02621 A2 disclose a method of forming flowable ribavirin particles, the process comprising: mixing ribavirin with at least one excipient to form a mixture; adding water to the mixture; forming the wet mixture into ribavirin containing particles; and drying the particles to form free flowing ribavirin containing particles.

Also WO 2004/096187 A1 and WO 03/39517 describe methods comprising wet granulation of ribavirin compositions. The recommended granulation liquid is water, and the tapped densities observed are above 0.6 g/cm$^3$.

It is an object of the present invention to provide an advantageous ribavirin preparation in the application form of a tablet, in particular a film tablet.

When investigating wet granulation methods for producing ribavirin formulations the inventors observed that ribavirin tablets produced by using water as a granulation liquid showed an increase of hardness after storage of more than 72 hours at room temperature by 70 N. An increase of hardness could have an uncontrollable influence on the dissolution of the active ingredient. Surprisingly, it was found that this "age-hardening" or "curing" was substantially reduced when using an aqueous alcohol solution as a granulation liquid. In addition, compositions with the obtained ribavirin granules exhibited favorable properties, namely a tapped density of less than 0.6 g/cm$^3$, in particular in the range from 0.45 g/cm$^3$ to 0.55 g/cm$^3$. This allows filling the tablet mould with a higher volume, resulting in lower mass variations of the tablet. In addition, it was unexpected that the pharmaceutical formulation according to the invention at the same time exhibited a good or excellent flowability while still having very good tabletting properties. That is, despite the rather poor processing properties of the active agent, such as a poor flowability, the present invention allows the preparation of tablets having desirable properties.

SUMMARY OF THE INVENTION

A first aspect of the present invention is a method for the manufacture of a pharmaceutical formulation, comprising the following steps:

a) blending ribavirin and at least one pharmaceutically acceptable excipient;
b) granulating the blend obtained from step a) in the presence of an aqueous granulation solution comprising from 5 wt.-% to 60 wt.-% of alcohol,
c) drying the granules obtained from step b) and
d) sieving and/or milling of dried granules
e) optionally adding one or more further pharmaceutically acceptable excipients to obtain the pharmaceutical formulation.

Optionally, step e) may further comprise milling or spheronizing the pharmaceutical formulation. The one or more further pharmaceutically acceptable excipients referred to in step e) may be co-milled with the dried granules. These excipients are usually excipients of the inner phase.

In one embodiment, the amount of alcohol in the granulation solution is in the range from 5 wt.-% to 20 wt.-%.

In another embodiment, the alcohol is selected from the group consisting of ethanol, isopropanol and combinations thereof.

In another embodiment, step a) comprises blending ribavirin with a filler, a binder and optionally with a disintegrant.

In another embodiment, step d) comprises adding an antiadherent, a lubricant, a glidant and optionally a disintegrant.

In another embodiment, the disintegrant is added in step a) and/or in step d).

In another embodiment, the antiadherent is colloidal silicon dioxide, the glidant is talc, and the lubricant is magnesium stearate.

In yet another embodiment, the filler is selected from the group consisting of pregelatinized starch, microcrystalline cellulose and combinations thereof, the binder is selected from the group consisting of pregelatinized starch, hypromellose and combinations thereof, and the disintegrant is crosscarmellose sodium.

A second aspect of the present invention is a pharmaceutical formulation obtainable by a method according to any one of the preceding claims, said formulation comprising

(a) ribavirin,
and at least one of the following excipients (b) to (d):
(b) a pharmaceutically acceptable filler,
(c) a pharmaceutically acceptable binder and
(d) a pharmaceutically acceptable disintegrant.

In another embodiment, the tapped density of the formulation is from 0.45 to 0.55 g/ cm$^3$.

In another embodiment, the pharmaceutical formulation comprises 40 to 65 wt.-% ribavirin, 20 to 30 wt.-% of the filler, 2 to 4 wt.-% of the disintegrant and 6 to 16 wt.-% of the binder.

In another embodiment, the pharmaceutical formulation further comprises one or more excipient(s) selected from the group consisting of pharmaceutically acceptable antiadherents, pharmaceutically acceptable lubricants, pharmaceutically acceptable glidants and combinations thereof.

In another embodiment, the pharmaceutical formulation comprises 0.5 to 1.5 wt.-% of the antiadherent, 2 to 4 wt.-% of the lubricant and 1 to 3 wt.-% of the glidant.

In another embodiment, the pharmaceutical formulation has a bulk density of less than 0.45 g/cm$^3$.

In another embodiment, the pharmaceutical formulation has a Hausner ratio of less than 1.3 and a CARR's index of 25% or less.

A third aspect of this invention is a method for the manufacture of a tablet comprising a) preparing a pharmaceutical formulation by a method according to the first aspect of the invention and compressing said pharmaceutical formulation into a tablet; or b) compressing a pharmaceutical formulation according to the second aspect of the invention into a tablet.

A fourth aspect of the invention is a method for the manufacture of a tablet, comprising the following steps:

a) blending ribavirin and at least one pharmaceutically acceptable excipient;
b) granulating the blend obtained from step a) in the presence of an aqueous granulation solution, comprising from 5 wt.-% to 60 wt.-% of alcohol, by fluid bed granulation,
c) drying the granules obtained from step b),
d) sieving and/or milling the dried granules obtained from step c),
e) optionally adding one or more further pharmaceutically acceptable excipients;
f) compressing the formulation obtained from step d) or e) into a tablet; and
g) optionally coating the tablet.

A fifth aspect of the invention is a tablet obtainable by a method according to the fourth aspect.
In one embodiment, the uncoated tablet is characterized by a hardness of 100 N to 200 N and/or by a friability of 0.1 to 1.0%, preferably 0.1 to 0.5 %
A sixth aspect of the invention is the use of a pharmaceutical formulation described herein for the manufacture of a tablet comprising ribavirin.
A seventh aspect of the invention is the use of a pharmaceutical formulation described herein for the manufacture of a medicament for the treatment of viral infections, preferably of HCV infections.
An eighth aspect of the invention is the pharmaceutical formulation described herein for treating viral infections, preferably HCV infections.

BRIEF DESCRIPTION OF THE DRAWINGS

Figure 1 shows the release profile of 200 mg Rebetol® capsules (reference example).

Figure 2 shows the release profile of 200 mg Copegus® film coated tablets (reference example).

Figure 3 shows the release profile of 200 mg film coated tablets prepared in Example 4.

Figure 4 shows the release profile of 200 mg film coated tablets prepared in Example 5.

Figure 5 shows the release profile of 400 mg film coated tablets prepared in Example 6.

DETAILED DESCRIPTION

*Ribavirin*

The ribavirin used in accordance with this invention is preferably substantially free of polymorphic forms of ribavirin, i.e. the polymorphic nature of ribavirin is not changed.
The relative amount of ribavirin and other components in the formulation will depend on the desired dosage. Suitable amounts of ribavirin may be in the range of from 40 wt.-% to 65 wt.-%, preferably from 50 wt.-% to 60 wt.-%, based on the total weight of the pharmaceutical formulation.
While the ribavirin formulations of this invention are described for 200 mg and 400 mg ribavirin strengths as tablets, other strengths of ribavirin, may be used without deviating from this invention.

*Excipients*

The ribavirin formulation of the present invention can be prepared with any excipient, such as at least any one of a filler, disintegrant, binder, etc., which is pharmaceutically acceptable and physically and chemically compatible with ribavirin.
Filler(s) or diluent(s) can be used in the formulation to provide bulk to the ribavirin composition. Examples of suitable fillers and diluents include lactose anhydrous, microcrystalline cellulose, starch, pregelatinized starch, modified starch, dibasic calcium phosphate dihydrate, calcium sulfate trihydrate, calcium sulfate dihydrate, calcium carbonate, lactose, dextrose, sucrose, mannitol, sorbitol, and their pharmaceutically acceptable salt or hydrate thereof, as appropriate. A combination of fillers and diluents can also be used. Preferred fillers and diluents are pregelatinized starch and microcrystalline cellulose. In a specific embodiment, the formulation of this invention does not contain lactose. The absence of lactose is preferred as such formulation can also be administered to patients suffering from lactose intolerance. In another specific embodiment, the formulation of this invention does not contain the inorganic fillers dibasic calcium phosphate dihydrate, calcium sulfate trihydrate, calcium sulfate dehydrate and calcium carbonate. This facilitates the preparation of formulations with lower tapped density.

The amount of filler in the compositions of the invention is preferably from about 0.5 to about 50 weight percent, preferably from about 2.5 to about 40 wt.-%, most preferably about 5 to about 25 wt.-%, based on the total weight of the pharmaceutical formulation.

Any disintegrant that is chemically and physically compatible with ribavirin can be used in the composition. Examples of suitable disintegrants include croscarmellose, sodium starch glycolate, corn starch, pregelatinized starches, polacrillin potassium, polyacrylates such as Carbopol, sodium carboxymethyl cellulose, potato starch, microcrystalline cellulose, crosslinked polyvinylpyrrolidone, magnesium aluminium silicate, bentonite, alginic acid and alginates, or their pharmaceutically acceptable salt or hydrate thereof, as applicable. A combination of disintegrants may also be used. The most preferred disintegrant is crosscarmellose sodium.

The amount of disintegrant in the compositions of the invention is preferably from about 0.5 to about 10 weight percent, based on the total weight of the ribavirin formulation, e.g., from about 1 to about 6 wt.-%. Preferably the amount of disintegrant is in the range from 2 to 4 wt.-%, most preferably it is about 3 to 3.5 wt.-%, based on the total weight of the ribavirin formulation.

Any pharmaceutically acceptable binder that is compatible with ribavirin can be used in the composition. Examples of suitable binders include starches, e.g., potato starch, wheat starch, corn starch; pregelatinized starch; gums such as gum tragacanth, acacia gum, and gelatine; microcrystalline cellulose, hydroxypropyl cellulose, hydroxyethyl cellulose, and hydroxypropylmethyl cellulose; and polyvinyl pyrrolidone, e.g., Povidone. Most preferred are pregelatinized starch and microcrystalline cellulose.

The amount of binder in the formulations of the invention is preferably from about 1 to about 30 weight percent, based on the total weight of the ribavirin formulation. Preferably the amount of binder is in the range from about 2 to about 25 wt.-%, more preferably from about 4 to about 22 wt.-%, most preferably it is from about 7 to bout 18 wt.-%, based on the total weight of the ribavirin formulation.

Other excipients that can be combined with ribavirin include hydroxypropyl cellulose, hydroxypropylmethyl cellulose, etc. In addition to excipients such as fillers, disintegrants, binders, etc. the ribavirin composition can also include a lubricant. Any pharmaceutically acceptable solid or liquid lubricant can be used in the composition, which is compatible with ribavirin. Typically the lubricant is used to enhance the flow and prevent sticking of the ribavirin composition. Suitable lubricants include magnesium stearate, calcium stearate, zinc stearate, talc, propylene glycol, polyethylene glycol, stearic acid, vegetable oil, sodium benzoate, sodium lauryl sulfate, magnesium lauryl sulfate, mineral oil, and polyoxyethylene monostearate as well as other salts and hydrates thereof. A combination of lubricants can also be used. The most preferred lubricant is magnesium stearate.

The amount of lubricant in the compositions of the invention is preferably from about 0.1 to about 5 weight percent, more preferably from about 0.5 to about 4 wt.-%, based on the total weight of the ribavirin composition.

Glidants enhance the flow of a powder mixture by reduction of interparticulate friction. In the present case, the glidant(s) preferably enhance the flow of the final composition (see below). Suitable glidants used in accordance with this invention include magnesium trisilicate, talc, colloidal silicon dioxide and combinations thereof. The amount of glidant is usually in the range from about 0.25 wt.-% to about 5 wt.-%, preferably it is in the range from about 0.5 wt.-% to about 3 wt.-%, based on the total weight of the ribavirin composition.

*Process of preparation*

The method of the invention includes the following steps:

　　a) blending ribavirin and at least one pharmaceutically acceptable excipient;
　　b) granulating the blend obtained from step a) in the presence of an aqueous granulation solution comprising from 5 wt.-% to 60 wt.-% of alcohol by fluid bed granulation,
　　c) drying the granules obtained from step b),
　　d) sieving and/or milling the dried granules obtained from step c), and
　　e) optionally adding one or more further pharmaceutically acceptable excipients to obtain the pharmaceutical formulation.

In step a) ribavirin is admixed with at least one excipient. Typically, ribavirin is first mixed with a filler and a binder. Also a disintegrant may be admixed in step a). In a first embodiment, step a) includes blending ribavirin with pregelatinized starch and microcrystalline cellulose. In a second embodiment, step a) includes blending ribavirin with pregelatinized starch, microcrystalline cellulose and crosscarmellose sodium.

In step b) the blend from step a) is mixed with a granulation liquid which is a mixture of water with alcohol. The expressions "granulation liquid", "granulation solution" and "wetting agent are used interchangeably herein.

The concentration of alcohol in the aqueous granulation liquid typically ranges from 5 wt.-% to 60 wt.-%, preferably it ranges from 8 wt.-% to 40 wt.-%, more preferably from 10 wt.-% to 20 wt.-%. The most preferred concentration of alcohol in the granulation liquid is about 10 wt.-%.

Preferred alcohols to be used in the granulation liquid include ethanol, isopropanol and combinations thereof. Most preferably, the granulation liquid is an aqueous solution comprising isopropanol and no other alcohols. In a special embodiment, the granulation solution consists of water and alcohol, preferably it consists of water and isopropanol. The above indicated amounts of alcohol in the granulation solution apply to this special embodiment *mutatis mutandis*.

The granulation liquid can already be added during step a), i.e. a granulation liquid can be already added when blending ribavirin with the one or more excipients in step a). It is also possible to first granulate one or more excipient (s) and then add the ribavirin to the granulation mixture. These embodiments are encompassed by steps a) and b) of the claimed method.

In one embodiment of the present invention, a ribavirin mixture can be granulated with granulation solution in an amount that is below, about equal to, or exceeds the amount of the dry mixture, e. g., a ribavirin blend can be granulated by adding granulation solution in an amount of about 50 wt.-%, 60 wt.-%, 70 wt.-%, 80 wt.-%, 90 wt.-%, 100wt.-%, or more, or any amount therebetween based on the dry ingredients of the mixture. In a preferred embodiment of the present invention, the amount of granulation solution added to granulate a ribavirin mixture is between about 100 wt.-% to about 180 wt.-% or between 130 wt.-% to about 170 wt.-%, based upon the dry ingredients.

The method of the invention also advantageously facilitates preparing ribavirin formulations having a narrow particle size distribution and a good content uniformity. In one aspect of the present invention, ribavirin particles are formed by sieving and/or milling a ribavirin composition.

By providing free flowing ribavirin particles having a narrow size distribution, ribavirin dosages can be manufactured from these compositions with reduced weight variation among the manufactured dosages. Further, since the particles have a high uniformity of ingredients, the manufactured dosages would also have a uniform content of ingredients. Such compositions facilitate controlling the release rate and its consistency in the preparation of immediate release and sustained release products.

In step c) granules prepared in the preceding step are dried. Drying is preferably performed using a fluid bed dryer or a tray dryer. Most preferably a fluid bed dryer is used at drying temperatures between 65 to 80 °C of inlet air and a corresponding product temperature of 25 to 55 °C.

In step d) the dried granules are sieved and/or milled. Sieving can be carried out by using a 0.5 to 1.5 mm sieve, e.g., by using a 1 mm sieve. Milling can be performed according to generally known techniques, e.g., using a sieving machine with a rasp sieve of 1 to 3.2 mm , e.g. by using a 2.0 mm sieve.

In step e) further pharmaceutically acceptable excipients may be added to the dried granules prepared in step c). Alternatively, if the dried granules are milled, the further excipients are added to the milled granules. Typical excipients added at this stage include lubricant, glidant, binder, antiadherent and disintegrant. Anti-adherents reduce sticking of the tablet to the die and punch. A suitable anti-adherent in accordance with this invention is collodidal silicon dioxide.

The pharmaceutical formulation obtained from step d) [in case step d) is not carried out] or from step e) is referred to herein as "final composition". The final composition of the invention can then be compressed into tablets by methods that are known *per se.* That is, the final composition is the pharmaceutical formulation prior to compression into tablets. The final composition is also known as "mass ready for tabletting".

*Properties of the pharmaceutical formulation*

A. Final composition

The final composition of this invention is characterized by a tapped density of less than 0.6 g/cm$^3$. The term "tap density" or "tapped density", as used herein, each denotes the measured mass of a powder attained at a limiting volume measured in a cylinder after being "tapped down", typically by a mechanical device; typically tap density is recorded as mass in grams divided by volume. The tap density is measured using a device described in the Ph. Eur. 6.0, Method 2.9.15. In difference to the conditions described therein the measurement of the tapped volume will be done only after 1250 stamps of the mechanical device instead of measurement after 500 and 1250 stamps or if necessary after 2500 stamps.

Preferably the tapped densities of the final composition is in the range of about 0.40 g/ cm$^3$ to about 0.55 g/ cm$^3$, more preferably in the range of 0.42 g/ cm$^3$ to about 0.53 g/ cm$^3$.

The bulk density of the final composition of the present invention typically ranges from 0.35 to 0.45 g/cm$^3$. Preferably, the bulk density is in the range from 0.36 to 0.43 g/cm$^3$. The bulk density is measured in accordance with the procedure described in Ph. Eur. 6.0, Method 2.9.15.

The CARR's index of the final composition of the invention may be 25 % or less, more preferably 22 % or less. Furthermore, the Hausner ratio of a final composition of the invention is preferably less than 1.35, more preferably less than 1.33, e.g. less than 1.3. The CARR's index can be determined as follows:

$$CARR's\ Index\ [\%]\ \ = \frac{tapped\ density - bulk\ density}{tapped\ density}\ \times 100\ \ \ [\%]$$

The Hausner ratio can be determined as follows:

$$Hausner\ ratio\ =\ \frac{tapped\ density}{bulk\ density}\ =\ \frac{bulk\ volume}{tapped\ volume}$$

The Hausner ratio and the CARR's index allow to predict flowability of a powder composition. The lower the number, the more free-flowing is the powder. An increase in the value is proportional to adhesion and friction properties of a powder, including (attractive) triboelectric charge.

It has been found by the present inventors that the formulation exhibits statisfactory flowability despite a relatively low tapped density. In particular, the final composition can be efficiently compressed into tablets. Alternatively, the granules can be filled into capsules or sachets.

B. Tablets

The hardness of the tablet cores is usually in the range from about 100 to 200 N, more preferably from 110 to 190 N. The hardness of the film-coated tablets usually ranges from 150 to 400 N, preferably from 180 to 350 N. Hardness of the film-coated tablets can be determined by methods known to somebody skilled in the art. The hardness of tablets can be determined according to the method described in Ph. Eur. 6.0 <2.9.8>. Unless stated otherwise, the hardness of tablets referred to herein means the "final hardness", i.e. the hardness after any "age-hardening" or "curing" has been substantially completed, and when no further siginificant change in hardness can be observed over time. Substantially, the increase in hardness following tabletting is completed within 4 days. That is, 96 hours after tabletting no significant change in hardness is usually observed anymore. In one embodiment, the above hardness values refer to determination 96 hours after tabletting or later. Generally, any parameters of the tablets of the invention refer to a determination at 96 hours after tabletting or later.

The increase in hardness of the tablets of the invention after tabletting is limited. Preferably, the increase in hardness within 96 hours after tabletting is less than 50 N, more preferably less than 40 N, still more preferably less than 30 N, most preferably less than 25 N. In a very similar embodiment, the increase in hardness within 120 hours after tabletting is less than 50 N, more preferably less than 40 N, still more preferably less than 30 N, most preferably less than 25 N.

The disintegration time of the film-coated tablets of the present invention may be 10 minutes or less, preferably 8 minutes or less, most preferably from 2 to 6 minutes. Disintegration times can be measured in vitro according to Ph. Eur. 6.0 <2.9.1>.

A particular advantage of the tablets of this invention is their advantageous dissolution profile. The tablets release a substantial portion of the active ingredient, ribavirin, within 20 or 30 minutes, determined according to Ph. Eur. 6.0 <2.9.3>, Apparatus 2, in 900 ml 0,1 HCl pH **1.2** at 37°C and 50 rpm. In a first embodiment, the tablets exhibit the following release of ribavirin: at least 20% within 5 minutes, at least 50% within 10 minutes, at least 75% within 15 minutes, and at least 90% within 20 minutes, determined according to Ph. Eur. 6.0 <2.9.3>, Apparatus 2, in 900 ml 0,1 HCl pH **1.2** at 37°C and 50 rpm.

In a second embodiment, the tablets exhibit the following release of ribavirin: 15 to 30% within 5 minutes, 50 to 70% within 10 minutes, 70 to 90% within 15 minutes, and at least 90% within 20 minutes, determined according to Ph. Eur. 6.0 <2.9.3>, Apparatus 2, in 900 ml 0,1 HCl pH **1.2** at 37°C and 50 rpm.

In a third embodiment, which is preferred, the tablets exhibit the following release of ribavirin: at least 80% within 5 minutes, at least 90% within 10 minutes, and at least 95% within 15 minutes, determined according to Ph. Eur. 6.0 <2.9.3>, Apparatus 2, in 900 ml 0,1 HCl pH **1.2** at 37°C and 50 rpm. Preferably, the ribavirin release profile is as follows: at least 90% within 5 minutes, at least 95% within 10 minutes, and at least 98% within 15 minutes, determined according to Ph. Eur. 6.0 <2.9.3>, Apparatus 2, in 900 ml 0,1 HCl pH **1.2** at 37°C and 50 rpm. The formulations shown in Examples 4 to 6 represent specific formulations according to this embodiment.

In a fourth embodiment, the tablets exhibit the following release of ribavirin: 40 to 60% within 5 minutes, 80 to 98% within 10 minutes, and 90 to 99% within 15 minutes, determined according to Ph. Eur. 6.0 <2.9.3>, Apparatus 2, in 900 ml 0,1 HCl pH **1.2** at 37°C and 50 rpm.

An important advantage of the tablets of the present invention, in particular of the above-mentioned third embodiment, is a very low variation in the release of ribavirin from tablet to tablet. The tablets of this embodiments are characterized by a very consistent and highly reproducible release profile. That is, the standard deviation in the release profile of ribavirin is low for the formulations of the present invention.

Unless indicated otherwise, dissolution profiles referred to herein are determined according to Ph. Eur. 6.0 <2.9.3>, Apparatus 2, in 900 ml 0,1 HCl pH **1.2** at 37°C and 50 rpm, $\lambda$=207 nm. Ribavirin is detected by measuring the absorption at a wavelength $\lambda$=207 nm.

The friability of the tablets cores usually ranges from 0.1 to 1.0 %, preferably from 0.1 to 0.5 %, determined using an apparatus described in Ph. Eur. 6.0 <2.9.7>. In difference to the conditions described therein the measurement of friability will be done after 500 rotations instead of 100 rotations.

The standard deviation of the mass of the tablet cores is advantageously not more than 2.2 %, preferably less than 1.5 %, more preferably less than 1 %

## Examples

Unless indicated otherwise, the parameters "tapped density" and "bulk density" were determined on the final composition prior to tabletting.

| Name | Function | example 1 | | example 2 | | example 3 | |
|---|---|---|---|---|---|---|---|
| | | mass per tablet [mg] | | | | | |
| Ribavirin | API | 200.0 | 400.0 | 200.0 | 400.0 | 200.0 | 400.0 |
| Starch | Filler | 55.2 | 110.4 | 55.2 | 110.4 | 55.2 | 110.4 |
| Cellulose, microcrystalline PH 101 (MCC) | Filler | 80.0 | 160.0 | 80.0 | 160.0 | 80.0 | 160.0 |
| Crosscarmellose sodium | Disintegrant | 16.0 | 32.0 | 16.0 | 32.0 | 16.0 | 32.0 |
| Talc | Glidant | 10.7 | 21.4 | 10.7 | 21.4 | 10.7 | 21.4 |
| Colloidal silicon dioxide | Antiadherent | 6.4 | 12.8 | 6.4 | 12.8 | 6.4 | 12.8 |
| Polyvinylpyrrolidone | Binder | 27.7 | 55.4 | 27.7 | 55.4 | 27.7 | 55.4 |
| Magnesium stearate | Lubricant | 4.0 | 8.0 | 4.0 | 8.0 | 4.0 | 8.0 |
| **Total** | | **400.0** | **800.0** | **400.0** | **800.0** | **400.0** | **800.0** |
| | | | | | | | |
| **Granulation liquid** | | | | | | | |
| Water | | 300.0 | 600.0 | - | - | - | - |
| Water-Isopropanol 90:10 | | - | - | 300.0 | 600.0 | - | - |
| Water-Isopropanol 60:40 | | - | - | - | - | 300.0 | 600.0 |
| | | | | | | | |

(continued)

| Granulation liquid | | | | | | |
|---|---|---|---|---|---|---|
| IPC[1]-Data of the cores: | | | | | | |
| Tapped density [g/cm$^3$] | | 0.39 | | 0.34 | | 0.36 | |
| Hardness [N] (measured as inprocess-control during manufacturing) - Average | | 150 | | 140 | | 145 | |
| Hardness [N] (measured between 4 and 10 days after tabletting) - Average | | 222 | | 166 | | 164 | |
| Tablet size and shape | | Diameter: 11 mm, radius of curvature: 10.5 mm | | Diameter: 12 mm, radius of curvature: 23 mm | | Diameter: 12 mm, radius of curvature: 23 mm | |
| Disintegration time [min.:sec.] | | 04:06 | | 03:27 | | 03:35 | |
| Friability [%] | | 0.33 | | 0.27 | | n.d. | |
| Standard deviation of mass | | 1.99 | | 0.61 | | 0.87 | |
| [1] IPC = "In process control". | | | | | | |

**Method of manufacture:**

The manufacture of tablets followed a standard wet granulation method. All excipients except magnesium stearate were sieved by use of a 1.0 mm sieve. Magnesium stearate was sieved by use of a 0.8 mm sieve. Ribavirin and the excipients except magnesium stearate were mixed in the fluid bed dryer for 10 minutes. Water or the water-isopropand mixture was sprayed onto granulate the mixture. Afterwards the granulate was dried. The resulting granulate was sieved by use of a 1.0 mm sieve. Magnesium stearate was added to the granulate, The blend was mixed for 2 min. The mixture was filled into a eccentric tabletting machine and compressed to tablets having a hardness of approx. 150 N. The 200 mg ribavirin containing tablets have a white aspect and a biconvex or biplane shape. The 400 mg ribavirin containing tablets have a white aspect and a oval shape.

**Results:**

Tablets of example 1 are especially characterized by a higher variability in tablet mass and a slightly longer disintegration time compared to tablets of example 2 and 3. The hardness of the cores of the tablets of example 1 is noticeably higher than the hardness of the tablets of example 2 and 3. The increase of hardness after 5 days of storage at room temperature was 72 N.

Tablets of example 2 showed an increase of hardness after 4 days of storage at room temperature of only 26 N, tablets of example 3 were characterized by an increase of hardness of 19 N after 4 days of storage at room temperature.

**Examples 4 - 6:**

**Formulation containing pregelatinized starch as binder and filler, granulation of API with excipients, Crosscarmellose in the inner and outer phase**

| | | example 4 | example 5 | example 6 |
|---|---|---|---|---|
| **Name** | **Function** | **200mg** | | **400mg** |
| **Inner phase (granules)** | | | | |
| Ribavirin | API | 200.0[1] | | 400.0 |
| Starch, pregelatinized | Filler | 15.0 | | 30.0 |
| Starch, pregelatinized | Binder | 45.0 | | 90.0 |
| Crosscarmellose sodium | Disintegrant | 6.0 | | 12.0 |
| Cellulose, microcrystalline PH 101 (MCC) | Filler | 78.0 | | 156.0 |
| **Granulation liquid** | | | | |
| Water | | 405.0 | | 910.0 |
| Isopropanol | | 45.0 | | 90.0 |
| | | | | |
| Crosscarmellose sodium | Disintegrant | 6.0 | | 12.0 |
| Colloidal silicon dioxide | Antiadherent | 3.0 | | 6.0 |
| Talc | Glidant | 10.0 | | 20.0 |
| Magnesium stearate | Lubricant | 7.00 | | 14.00 |
| **Total Cores:** | | **370.0** | | **740.0** |
| **Coat:** | | | | |
| Hypromellose | Film forming agent | 9.6 | | 19.19 |
| PEG 6000 | Plasticiser | 0.96 | | 1.92 |
| Talc | Separating agent | 1.92 | | 3.84 |
| Ferric oxide red | Colouring agent | 0.003 | | 0.36 |
| Titan dioxide | Colouring agent | 2.517 | | 4.69 |
| **Total film coated tablets:** | | **385.0** | | **770.0** |
| | | | | |
| **IPC-Data[2]:** | | | | |
| Bulk density [g/cm$^3$] | | 0.39 | 0.40 | 0.41 |
| Tapped density [g/cm$^3$] | | 0.51 | 0.47 | 0.51 |
| CARR's-Index | | 25 | 14 | 21 |

(continued)

| Name | Function | example 4 | example 5 | example 6 |
| --- | --- | --- | --- | --- |
| | | 200mg | | 400mg |
| Hausner-ratio | | 1.33 | 1.17 | 1.26 |
| Hardness [N] cores | | 110 - 206 | 111 - 137 | 153 - 195 |
| Hardness [N] film coated tablets | | 198-269 | 180 - 225 | 279 - 336 |
| Tablet size and shape | | 14.3 x 6.9, oval | 14.3 x 6.9, oval | 18 x 8, oval |
| Disintegration time | | | | |
| [min.:sec.] film coated | | 8:31 | 5:43 | 5:03 |
| tablets | | | | |
| Friability [%] cores | | 0.15 | 0.01 | 0.06 |
| Standard deviation of mass | | 2.1 | 0.62 | 1.2 |
| [1] The ribavirin used in example 4 had a lower particle size than the ribavirin used in example 5 [2] IPC = "In process control". | | | | |

Method of manufacture:

Ribavirin is premixed for 10 min using a tumble mixer type Rhönrad with microcrystalline cellulose, filler pregelatinized starch and croscarmellose sodium and then transferred to the fluid-bed granulator. A preheating step in the fluid-bed granulator follows.

The binder pregelatinized starch is dissolved in the granulation liquid and sprayed onto the mixture. After granulation step the granules are dried in the fluid-bed granulator.
The excipients of the outer phase except the glidant are added and mixed on a tumble mixer type Rhönrad for 5 min. The glidant is added and the blend is mixed again for 2 min.
The blend is then pressed to tablets of the desired properties. The last step consists of the coating of the tablets.
The colouring agents are dispersed in water. The film forming agent is dissolved in water. The dispersion of the colouring agents is added to the solution of the film former and this dispersion is sprayed onto the tablets.

**Example 7:**

**Formulation containing pregelatinized starch as binder and filler, granulation of API with excipients, Crosscarmellose only in the outer phase**

| Name | Function | example 7 | |
| --- | --- | --- | --- |
| | | 200mg | 400mg |
| **Inner phase (granules)** | | | |
| Ribavirin | API | 200.0 | 400.0 |
| Starch, pregelatinized | Filler | 15.0 | 30.0 |
| Starch, pregelatinized | Binder | 45.0 | 90.0 |
| Cellulose, microcrystalline PH 101 (MCC) | Filler | 78.0 | 156.0 |
| **Granulation liquid** | | | |
| Water | | 405.0 | 910.0 |
| Isopropanol | | 45.0 | 90.0 |

(continued)

| Outer phase | | | |
|---|---|---|---|
| Crosscarmellose sodium | Disintegrant | 12.0 | 24.0 |
| Colloidal silicon dioxide | Antiadherent | 3.0 | 6.0 |
| Talc | Glidant | 10.0 | 20.0 |
| Magnesium stearate | Lubricant | 7.00 | 14.00 |
| **Total Cores:** | | **370.0** | **740.0** |
| | | | |
| **Coat:** | | | |
| Hypromellose | Film forming agent | 9.6 | 19.19 |
| PEG 6000 | Plasticiser | 0.96 | 1.92 |
| Talc | Separating agent | 1.92 | 3.84 |
| Ferric oxide red | Colouring agent | 0.003 | 0.36 |
| Titan dioxide | Colouring agent | 2.517 | 4.69 |
| **Total film coated tablets:** | | **385.0** | **770.0** |
| Bulk density [g/cm$^3$] | | 0.372 | |
| Tapped density [g/cm$^3$] | | 0.453 | |
| CARR's-Index | | 18 | |
| Hausner ratio | | 1.22 | |
| | | **example 7** | |
| **Name** | **Function** | **200mg** | **400mg** |
| Hardness [N] cores | | 158 - 232 | |
| Tablet size and shape | | 14.3 x 6.9, oval | |
| Friability [%] cores | | 0.27 | |
| Standard deviation of mass | | 0.5 | |

Method of manufacture:

Ribavirin is premixed for 10 min using a tumble mixer type Rhönrad with microcrystalline cellulose, filler pregelatinized starch and crosscarmellose sodium and then transferred to the fluid-bed granulator. A preheating step in the fluid-bed granulator follows.

The binder pregelatinized starch is dissolved in the granulation liquid and sprayed onto the mixture. After granulation step the granules are dried in the fluid-bed granulator.
The excipients of the outer phase except the glidant are added and mixed on a tumble mixer type Rhönrad for 5 min. The glidant is added and the blend is mixed again for 2 min.
The blend is then pressed to tablets of the desired properties. The last step consists of the coating of the tablets.
The colouring agents are dispersed in water. The film forming agent is dissolved in water. The dispersion of the colouring agents is added to the solution of the film former and this dispersion is sprayed onto the tablets.

**Examples 8 - 10:**

**Formulation containing pregelatinized starch as binder and filler, granulation of API with excipients, Crosscarmellose in the inner and outer phase, Hypromellose as additional excipient in the inner phase**

| Name | Function | example 8 3,5 mg HPMC 200mg | example 9 7,5 mg HPMC 200mg | example 10 10 mg HPMC 200mg |
|---|---|---|---|---|
| **Inner phase (granules)** | | | | |
| Ribavirin | API | 200.0 | 200.0 | 200.0 |
| Starch, pregelatinized | Filler | 15.0 | 15.0 | 15.0 |
| Starch, pregelatinized | Binder | 45.0 | 45.0 | 45.0 |
| Crosscarmellose sodium | Disintegrant | 6.0 | 6.0 | 6.0 |
| Hypromellose | Binder | 3.5 | 7.5 | 10 |
| Cellulose, microcrystalline PH 101 (MCC) | Filler | 78.0 | 78.0 | 78.0 |
| **Granulation liquid** | | | | |
| Water | | 405.0 | 405.0 | 405.0 |
| Isopropanol | | 45.0 | 45.0 | 45.0 |
| **Outer phase** | | | | |
| Crosscarmellose sodium | Disintegrant | 6.0 | 6.0 | 6.0 |
| Colloidal silicon dioxide | Antiadherent | 3.0 | 3.0 | 3.0 |
| Talc | Glidant | 10.0 | 10.0 | 10.0 |
| Magnesium stearate | Lubricant | 7.00 | 7.00 | 7.00 |
| **Total Cores:** | | **373.5** | **377.5** | **380.0** |
| **Coat:** | | | | |
| Hypromellose | Film forming agent | 9.6 | 9.6 | 9.6 |
| PEG 6000 | Plasticiser | 0.96 | 0.96 | 0.96 |
| Talc | Separating agent | 1.92 | 1.92 | 1.92 |
| Ferric oxide red | Colouring agent | 0.003 | 0.003 | 0.003 |
| Titan dioxide | Colouring agent | 2.517 | 2.517 | 2.517 |
| **Total film coated tablets**: | | **388.5** | **392.5** | **395.0** |
| | | | | |
| **IPC-Data:** | | | | |
| Bulk density [g/cm$^3$] | | 0.381 | 0.422 | 0.362 |
| Tapped density [g/cm$^3$] | | 0.457 | 0.503 | 0.422 |

(continued)

| IPC-Data: | | | | |
|---|---|---|---|---|
| CARR's-Index | | 17 | 16 | 14 |
| Hausner ratio | | 1.20 | 1.19 | 1.17 |
| Hardness [N] cores | | 162 - 189 | 109 - 132 | 105 - 140 |
| Hardness [N] film coated tablets | | 196 - 236 | 180 - 235 | 174 - 232 |
| Tablet size and shape | | 14.3 x 6.9, oval | 14.3 x 6.9, oval | 14.3 x 6.9, oval |
| Disintegration time [min.:sec.] film coated tablets | | 12:31 | 16:29 | 10:57 |
| Friability [%] cores | | 0.27 | 0.17 | 0.18 |
| Standard deviation of mass | | 0.53 | 0.84 | 1.7 |

Method of manufacture:

Ribavirin is premixed for 10 min using a tumble mixer type Rhönrad with microcrystalline cellulose, filler pregelatinized starch and crosscarmellose sodium and then transferred to the fluid-bed granulator. A preheating step in the fluid-bed granulator follows.

The binder pregelatinized starch is dissolved in the granulation liquid and sprayed onto the mixture. After granulation step the granules are dried in the fluid-bed granulator.

The excipients of the outer phase except the glidant are added and mixed on a tumble mixer type Rhönrad for 5 min. The glidant is added and the blend is mixed again for 2 min.

The blend is then pressed into tablets of the desired properties. The last step consists of the coating of the tablets.

The colouring agents are dispersed in water. The film forming agent is dissolved in water. The dispersion of the colouring agents is added to the solution of the film former and this dispersion is sprayed onto the tablets.

Example 11

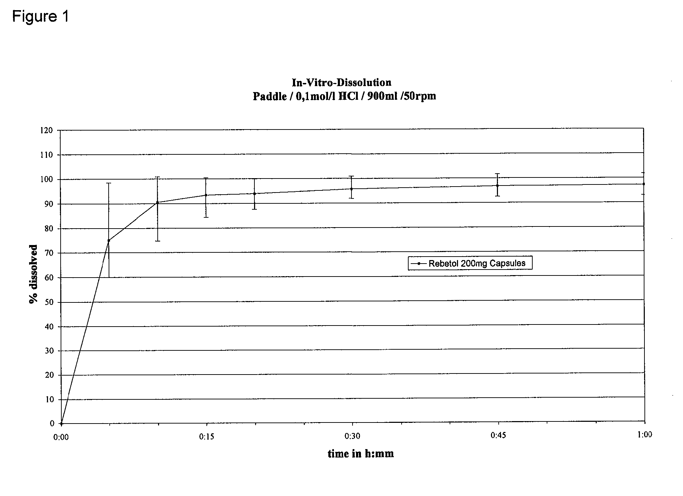

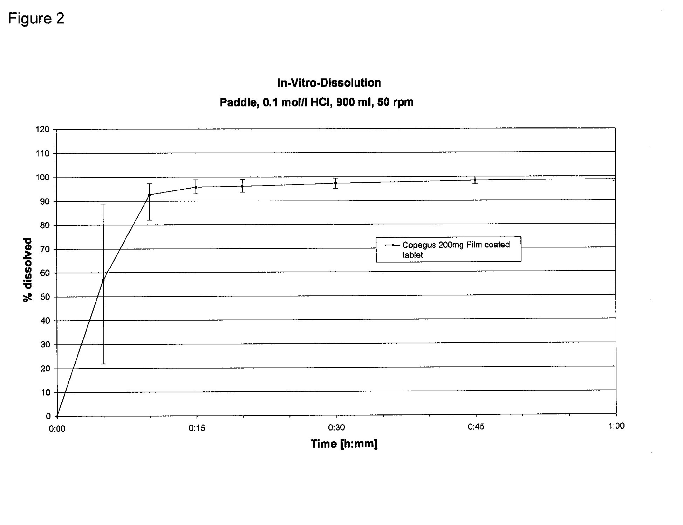

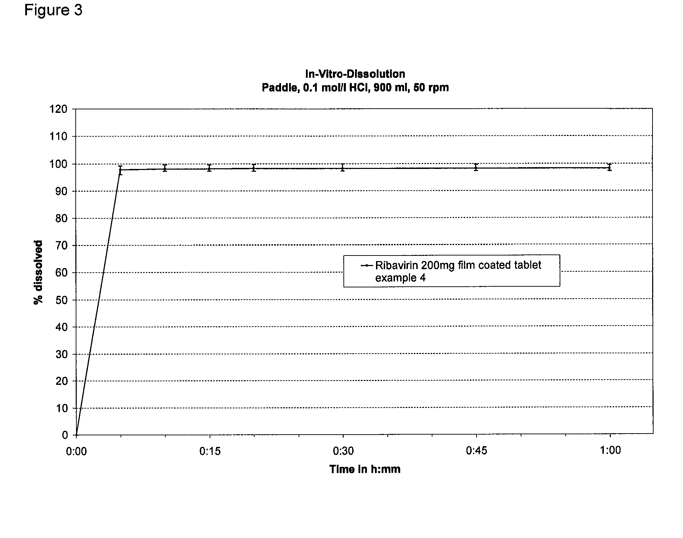

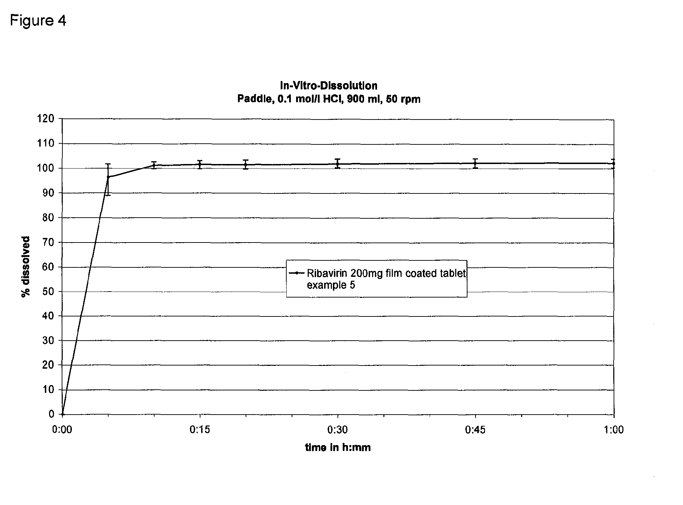

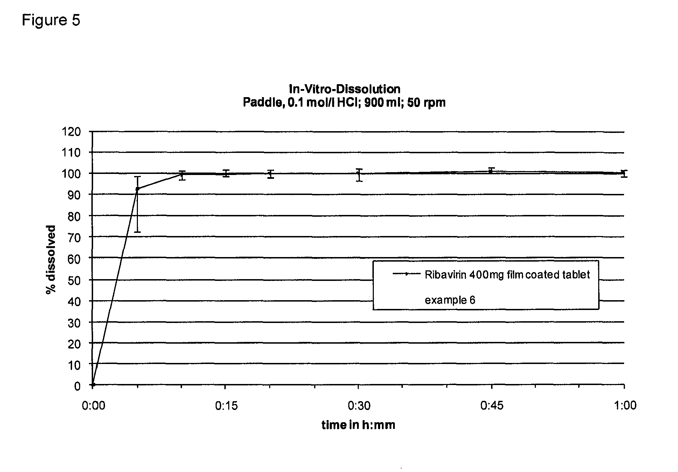

The release profiles of the tablets of examples 4, 5 and 6 have been determined and compared to the release profiles of two currently marketed products (Copegus® 200 mg film coated tablets and Rebetol® 200 mg capsules). The release profiles were determined in accordance with Ph. Eur. 6.0 <2.9.3>, Apparatus 2, in 900 ml 0,1 HCl pH **1.2** at 37°C and 50 rpm, λ=207 nm. Results are depticted in Figures 1 to 5.

As can be seen, the formulations of the present invention show a much faster release of ribavirin than the reference products Rebetol® and Copegus®.

**Claims**

1.  A method for the manufacture of a pharmaceutical formulation, comprising the following steps:

    a) blending ribavirin and at least one pharmaceutically acceptable excipient;
    b) granulating the blend obtained from step a) in the presence of an aqueous granulation solution comprising from 5 wt.-% to 60 wt.-% of alcohol,
    c) drying the granulate obtained from step b)
    d) sieving and/or milling the dried granules obtained from step c), and
    e) optionally adding one or more further pharmaceutically acceptable excipients to obtain the pharmaceutical formulation.

2.  The method of claim 1, wherein the amount of alcohol in the granulation solution is in the range from 5 wt.-% to 20 wt.-%.

3. The method of claim 1, wherein the alcohol is selected from the group consisting of ethanol, isopropanol and combinations thereof.

4. A pharmaceutical formulation obtainable by a method according to any one of the preceding claims, said formulation comprising:

   (a) ribavirin, and
   at least one of the following excipients (b) to (d):
   (b) a pharmaceutically acceptable filler,
   (c) a pharmaceutically acceptable binder and
   (d) a pharmaceutically acceptable disintegrant.

5. The pharmaceutical formulation according to claim 4, wherein said formulation has a tapped density in the range of 0.45 to 0.55 g/cm$^3$.

6. The pharmaceutical formulation according to claim 4 or 5, comprising 40 to 65 wt.-% ribavirin, 0 to 30 wt.-% of the filler, 2 to 4 wt.-% of the disintegrant and 6 to 16 wt.-% of the binder.

7. The pharmaceutical formulation according to any one of claims 4 to 6, further comprising a pharmaceutically acceptable antiadherent, a pharmaceutically acceptable lubricant and pharmaceutically acceptable glidant.

8. The pharmaceutical formulation according to claim 7, comprising 0.5 to 1.5 wt.-% of the antiadherent, 2 to 4 wt.-% of the lubricant and 1 to 3 wt.-% of the glidant.

9. The pharmaceutical formulation according to any one of claims 4 to 8, having a bulk density of less than 0.45 g/cm$^3$.

10. A method for the manufacture of a tablet comprising:

    a) preparing a pharmaceutical formulation by a method according to any one of claims 1 to 3 and compressing said pharmaceutical formulation into a tablet; or
    b) compressing a pharmaceutical formulation according to any one of claims 4 to 9 into a tablet.

11. A method for the manufacture of a tablet, comprising the following steps:

    a) blending ribavirin and at least one pharmaceutically acceptable excipient;
    b) granulating the blend obtained from step a) in the presence of an aqueous granulation solution comprising from 5 wt.-% to 60 wt.-% of alcohol,
    c) drying the granulate obtained from step b),
    d) sieving and/or milling the dried granules obtained from step c),
    e) optionally adding one or more further pharmaceutically acceptable excipients;
    f) compressing the formulation obtained from step d) or e) into a tablet; and
    g) optionally coating the tablet.

12. A tablet obtainable by a method according to claim 11.

13. A tablet comprising ribavirin and one or more pharmaceutically acceptable excipients, **characterized in that** the hardness of the tablet after tabletting increases by less than 50 N within 120 hours after tabletting.

14. The tablet of claim 12 or 13, **characterized by** a hardness of 100 N to 200 N, determined at 120 hours after tabletting.

15. The tablet of any one of claims 12 to 14, having a friability from 0.1 to 0.5%.

16. The use of a pharmaceutical formulation according to any one of claims 4 to 9 for the manufacture of a tablet, capsule or sachet comprising ribavirin.

17. The use of a pharmaceutical formulation according to any one of claims 4 to 9 for the manufacture of a medicament for the treatment of viral infections, in particular of HCV infections.

18. The pharmaceutical formulation according to any one of claims 4 to 9 or the tablet according to any one of claims 12 to 15 for treating viral infections, in particular HCV infections.

Figure 1

In-Vitro-Dissolution
Paddle / 0,1mol/l HCl / 900ml /50rpm

Figure 2

In-Vitro-Dissolution

Paddle, 0.1 mol/l HCl, 900 ml, 50 rpm

Figure 3

In-Vitro-Dissolution
Paddle, 0.1 mol/l HCl, 900 ml, 50 rpm

Figure 4

In-Vitro-Dissolution
Paddle, 0.1 mol/l HCl, 900 ml, 50 rpm

Ribavirin 200mg film coated tablet example 5

Figure 5

**In-Vitro-Dissolution**
**Paddle, 0.1 mol/l HCl; 900 ml; 50 rpm**

Europäisches
Patentamt

European
Patent Office

Office européen
des brevets

# EUROPEAN SEARCH REPORT

Application Number

EP 09 17 0031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 03/053450 A (BIOPARTNERS GMBH [CH]; SOBEL CORNELIUS [DE]; HUBER GERALD [DE]) 3 July 2003 (2003-07-03) | 1-18 | INV. A61K9/16 A61K9/20 |
| X | * page 9 * | 4,12, 16-18 | A61K9/28 A61K9/48 A61K9/26 |
| Y | WO 2004/026261 A (THREE RIVERS PHARMACEUTICALS L [US]; KERRISH DONALD J [US]; BERGERON J) 1 April 2004 (2004-04-01) | 1-18 | A61K9/32 A61K31/7056 |
| X | * paragraph [0031]; example 1; tables 3,4 * | 4,12, 16-18 | |
| Y | WO 03/053399 A (PFIZER PROD INC [US]; FERGIONE MICHAEL BRUCE [US]; JOHNSON BARBARA ALI) 3 July 2003 (2003-07-03) * example 3; tables 3,3A * | 1-18 | |

TECHNICAL FIELDS SEARCHED (IPC)

A61K

The present search report has been drawn up for all claims

| Place of search | Date of completion of the search | Examiner |
|---|---|---|
| Munich | 17 December 2009 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 09 17 0031

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2009

| Patent document cited in search report | | Publication date | Patent family member(s) | | Publication date |
|---|---|---|---|---|---|
| WO 03053450 | A | 03-07-2003 | AT | 290387 T | 15-03-2005 |
| | | | AU | 2002229678 A1 | 09-07-2003 |
| | | | CA | 2470342 A1 | 03-07-2003 |
| | | | DE | 50105577 D1 | 14-04-2005 |
| | | | EP | 1455801 A1 | 15-09-2004 |
| | | | ES | 2238497 T3 | 01-09-2005 |
| | | | JP | 4221299 B2 | 12-02-2009 |
| | | | JP | 2006502961 T | 26-01-2006 |
| | | | MX | PA04006159 A | 31-03-2005 |
| | | | NZ | 533808 A | 24-03-2005 |
| | | | US | 2005123612 A1 | 09-06-2005 |
| WO 2004026261 | A | 01-04-2004 | AU | 2003275123 A1 | 08-04-2004 |
| WO 03053399 | A | 03-07-2003 | AU | 2002353316 A1 | 09-07-2003 |
| | | | BR | 0215175 A | 28-12-2004 |
| | | | CA | 2470055 A1 | 03-07-2003 |
| | | | CN | 1606433 A | 13-04-2005 |
| | | | EP | 1455757 A2 | 15-09-2004 |
| | | | JP | 2005515212 T | 26-05-2005 |
| | | | MX | PA04003027 A | 05-07-2004 |
| | | | NZ | 532063 A | 31-03-2006 |
| | | | PA | 8562101 A1 | 04-02-2005 |
| | | | RU | 2283651 C2 | 20-09-2006 |
| | | | ZA | 200402586 A | 01-04-2005 |

For more details about this annex : see Official Journal of the European Patent Office, No. 12/82

**EP 2 295 037 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6337090 B
- CA 2135669
- US 20030104050 A1
- EP 991415 B1
- EP 1455801 A
- US 7538094 B2
- WO 200402621 A2
- WO 2004096187 A1
- WO 0339517 A